# EUROPEAN PATENT SPECIFICATION

(11) **EP 4 531 711 B1**
(45) Date of publication and mention of the grant of the patent: **25.11.2026**
(21) Application number: 23730085.0
(22) Date of filing: 31.05.2023
(51) Int. Cl.: A61B 17/17, A61B 17/80

(54) **ORTHOPAEDIC PLATE AND JIG**
ORTHOPÄDISCHE PLATTE UND SPANNVORRICHTUNG
PLAQUE ORTHOPÉDIQUE ET GABARIT

(30) Priority: 01.06.2022 GB 202208117
(43) Date of publication of application: 09.04.2025
(73) Proprietor: AT Ortho Limited, Clifton, Bristol BS8 2AL (GB)
(72) Inventor: ATKINS, Roger, Bristol BS8 2AL (GB); TUKE, Michael Antony, Guildford GU1 3TF (GB); TAYLOR, Andrew Clive, Chichester, West Sussex PO18 0AW (GB)
(74) Representative: Mewburn Ellis LLP
(86) International application number: PCT/EP2023/064603
(87) International publication number: WO 2023/232902

(56) References cited:
- US-A1- 2015 080 967
- US-A1- 2015 105 779
- US-A1- 2018 317 986
- US-A1- 2018 360 477
- US-B2- 11 197 682

## Description

### Technical Field

The present disclosure relates to an orthopaedic assembly comprising an orthopaedic plate and a jig for securing an orthopaedic plate to a bone. In particular, the present disclosure relates to an orthopaedic assembly which may be used, in combination with relevant tools/fasteners, to fix an orthopaedic plate to a patient's bone e.g. to a patient's tibia. In addition, the present disclosure relates to methods (not claimed) of fixing ar orthopaedic plate to a bone.

### Background

It is known to fix an orthopaedic plate against a patient's bone, e.g. against a patient's tibia, using bone screws in order to reduce and stabilise a fracture within the bone to allow bone healing. The orthopaedic plate is positioned and fixed internally within the patient's limb during orthopaedic surgery optionally with the aid of a jig which is connected to the plate. Such fixations are employed in many sorts of surgery, including fracture treatment, treatment of ununited bones and bone divisions for the correction of deformity. In the known plate and jig assemblies, the jig support typically extends parallel to the plate, facing and connected to a surface of the plate which, in use faces away from the bone. The plate is inserted against a surgically accessible surface of the bone, which may be superficial underneath the skin, with the plate interposed between the bone and the jig support. Once the plate is in position, fasteners such as bone screws are inserted through bores in the jig support and plate into the bone (from the surgically accessible (e.g. superficial) aspect of the bone) such that the heads of the fasteners engage the plate at the surface of the plate facing the jig support (i.e. facing away from the bone). The bores in the jig support and the plate are aligned to ensure the correct alignment between the fasteners and plate bores.

One problem with the known orthopaedic plates arises as a result of their positioning on the surgically accessible surface of the bone, which may be superficial, just under the patient's skin. This can lead to skin breakdown over the plate which, in extreme cases can lead to limb amputation. Positioning of the plate on a deep surface of the bone i.e. where the bone is interposed between the plate and patient's skin over the superficial surface of the bone should avoid this skin breakdown but these deep bone surfaces are often not accessible by direct surgical exposure. A second issue is that some surgical actions optimally require a plate to be located on a surgically inaccessible or deep bone surface. Such plate positioning is simply not technically feasible using the known plate and jig assemblies. The surgical exposure necessary to position a plate directly on a deep surface of the bone and then to pass the fasteners from deep within the limb, through the plate into the deep surface of the bone would be too great with a significant risk of damage to the nerves and arteries within the limb. It is often possible to place a plate on a bone surface that is not directly surgically accessible by using a short surgical incision at one end of the bone but the fastenings cannot be placed through the plate into the bone in the standard manner detailed above.

The present disclosure has been devised in light of the above considerations with a view to provide a plate and jig assembly that that be used to locate and secure a plate on a deep surface of a bone e.g. the posterior surface of the tibia whilst reducing surgical exposure and risk of nerve/artery damage.

US 2015/080967 A1 proposes a system for making a bone repair. US 2015/105779 A1 proposes a combined intramedullary and extramedullary surgical aiming system and method. US 2018/360477 A1 proposes patient-specific bridging plates.

### Summary

The scope of the invention is defined by the appended claims. The methods of transosseous plating disclosed herein are not within the scope of the invention.

In a first aspect, there is provided an assembly for securing an orthopaedic plate to a bone according to claim 2.

By providing an assembly where an orthopaedic plate and jig define, between them, an elongated (longitudinally-extending) space for receiving a bone requiring fixing (e.g. an axially extending fracture portion of bone such as a tibia), the plate can be inserted into the limb (e.g. leg) through a small incision with the elongate shaft positioned against the deep surface of the bone whilst the elongate jig support remains external to the limb such that, in use, the jig can guide relevant fasteners through the bone (from the surgically accessible or superficial surface) and into engagement within the plate bores. Thus, in use, the orthopaedic plate is internal to the limb, the bone contact surface of the elongate shaft contacts the bone, and the elongate jig support is external to the limb. The heads of the fasteners will sit against the surgically accessible surface of the bone. This "transosseous plating" allows positioning of the plate deep within the limb in a surgically inaccessible site and thus does not cause skin breakdown whilst also avoiding the need for any significant surgical exposure and reducing the risk of nerve/artery damage during fastener insertion due to the absence of nerves and arteries proximal the superficial surface of the bone.

Optional features will now be set out. These are applicable singly or in any combination with any aspect or embodiment.

The bone-receiving space between the bone contact surface and the jig support is an unobstructed space prior to insertion into the patient's limb i.e. there are no obstructions extending transversely across the space between the bone contact surface and the jig support so that the bone can positioned within the space without meeting impedance from any part of the assembly. Post insertion (in use), the bone together with a portion of the patient's limb is received within the longitudinally-extending bone-receiving space such that a portion of the limb's skin is interposed between the orthopaedic plate and the jig support, and between the bone and the jig support.

At least one of the array of plate bores is a threaded plate bore.

The or each threaded plate bore may be a conical threaded plate bore having a threaded conical portion tapering from a maximum diameter proximal the bone contact surface to a minimum diameter distal the bone contact surface.

The threaded plate bore(s) e.g. the conical threaded plate bore(s) may (each) comprise a tapered thread having more than one (e.g. two) starts.

The orthopaedic plate of the first aspect may have any one or more of the features described below in relation to the second aspect.

In a second aspect, there is provided an orthopaedic plate for securing to a bone according to claim 1.

The orthopaedic plate of the second aspect is designed for transosseous plating and the plate bores are for receiving the tip portion of a fastener from the bone contact surface. Thus the tapering of the bore from a maximum diameter at the bone contact surface to a reduced diameter provides for the locking of the fastener as the tip portion advances within the conical plate bore from the bone contact surface.

The conical threaded plate bore(s) may (each) comprise a tapered thread having more than one (e.g. two) starts.

In some embodiments, at least one conical plate bore comprises a cylindrical portion (having the maximum diameter) proximal the bone contact surface with a step/shoulder between the cylindrical portion and the conical portion which tapers in diameter away from the cylindrical portion. This cylindrical portion is configured to accommodate a drill bit protruding from the bone without damage to the conical portion. This is discussed in more detail below.

In some embodiments of the first or second aspect, the plate comprises a head portion at an axial end of the elongate shaft. The head portion may have an enlarged width in a direction perpendicular to the elongate shaft and perpendicular to a depth direction between the bone contact surface and the opposing surface. The head portion may comprise at least one head bore. For example, the head portion may comprise a plurality of head bores. The or each head bore may be a threaded head bore. The head bores are provided for each receiving a respective head fastener for affixing the head portion to the enlarged head of the bone e.g. to the enlarged head of the tibia.

The head portion may extend out of the plane of the elongate shaft. For example, the head portion may extend so that there is an angle of greater than 180 degrees between head portion and the bone contact surface and an angle of less than 180 degrees between the head portion and the opposing surface. This allows the elongate shaft to sit against the deep surface of the bone shaft whilst the head portion accommodates the (deep surface of the) enlarged head of the bone.

In the assembly of the first aspect, the head portion may extend at an angle away from the jig support. In the absence of the jig support (e.g. in the second aspect), the angular relationship between the head portion and the surface of the plate may be used to distinguish between the bone contact surface and the opposing surface.

The head portion may comprise a tool receiving portion for receiving an insertion tool. For example, the tool receiving portion may be a recessed portion e.g. a recess or channel for receiving the tool. This facilitates insertion of the plate against the deep surface of the bone as the tool can be engaged in the recessed portion and used to push the elongate shaft within the patient's limb so that the elongate shaft lies against the bone shaft (and the head portion is seated against the enlarged head of the bone).

Insertion of the plate may additionally or alternatively be facilitated by the provision of an insertion portion on the elongate shaft terminating at an insertion axial end (at the opposite axial end to the head portion where provided). The insertion portion may have a rounded insertion axial end. It may be rounded in the plane of the elongate shaft and/or in a plane transecting the elongate plate support in the depth direction between the bone contact surface and the opposing surface.

The insertion portion may taper along the length of the insertion portion to a reduced depth (i.e. to a reduced spacing between the bone contact surface and the opposing surface of the elongate shaft). This may be achieved by tapering of the opposing surface towards the bone contact surface of the elongate shaft.

In the assembly of the first aspect, the opposing surface of the elongate shaft may taper towards the jig support. In the absence of the jig support (e.g. in the second aspect), the tapering of the surface of the plate may be used to distinguish between the surface which is configured to face the bone and the (tapering) opposing surface.

In some embodiments, the opposing surface of the elongate shaft and/or the head portion (i.e. the surfaces configured to face away from the bone) may have a chamfered edge. In the absence of the jig support (e.g. in the second aspect), the location of the chamfered edge may be used to distinguish between the surface which is configured to face the bone and the opposing surface (with the chamfered edge).

The array of plate bores may form a row of bores aligned with the axial extension (length) of the elongate shaft. The plate bores may be aligned with the axis of the elongate shaft. The plate bores may be substantially equally spaced along the length of the elongate shaft.

In some embodiments, the array of plate bores comprises one or more insertion portion bores at or proximal the insertion axial end, wherein at least one of the insertion portion bores is a cylindrical threaded bore. The threaded bore is configured to receive an alignment screw, the purpose of which will be described later.

The jig may comprise a connector portion extending between and rigidly connecting the jig support and the orthopaedic plate. The connector portion is provided to maintain a fixed spatial relationship between the jig support and orthopaedic plate. That is, the connector portion is provided to maintain the elongate jig support and the orthopaedic plate in parallel alignment such that the spacing between the jig support and orthopaedic plate is constant along their respective longitudinal axes. The connector portion may further maintain the elongate jig support and the orthopaedic plate in fixed alignment such that orthopaedic plate overlies the elongate jig support along its longitudinal axis (or vice versa). Conveniently, when the jig support comprises an array of jig bores as discussed below, this can ensure correct alignment of the plate bores with the jig bores. The connector portion is removably connected to the orthopaedic plate. It may be removably connected to the opposing surface of the orthopaedic plate. It may be removably connected to the head portion of the orthopaedic plate e.g. to the opposing surface of the head portion.

The connector portion may comprise a connector body. The connector body may comprise a first socket proximal the orthopaedic plate, the first socket housing a fixing element for removably fixing the connector body to the orthopaedic plate. The opposing surface of the head portion of the plate may comprise locator indents for receiving locator tabs provided on the fixing element. The fixing element may comprise a fixing bore for receiving a connector fixing fastener for removably fixing the fixing element in one of the head bores.

The jig of the first aspect may have any one or more of the features described below in relation to the first reference example below.

In a first reference example, there is provided a jig for securing an orthopaedic plate to a bone, the jig comprising an elongate jig support and a connector portion that curves or bends in a plane that is substantially perpendicular to and transects the elongate axis of the jig support.

In the first aspect, the connector portion also curves/bends in a plane that is substantially perpendicular to and transects the elongate axis of the elongate shaft of the orthopaedic plate.

The jig support comprises an internal surface (which, in use, faces the patient's limb) and an opposing external surface. In the assembly of the first aspect, the internal surface faces the bone contact surface of the elongate shaft of the orthopaedic plate i.e. the longitudinally-extending bone-receiving space is defined between the bone contact surface of the orthopaedic plate and the internal surface of the jig support. When the jig comprises the connector portion discussed above, the connector portion is provided to maintain a constant spacing between the internal surface of the jig support and the bone contact surface of the orthopaedic plate along the longitudinal axes of the jig support and the orthopaedic plate. The connector portion may further maintain the elongate jig support and the orthopaedic plate in fixed alignment such that the bone contact surface of the orthopaedic plate overlies the internal surface of the jig support along its longitudinal direction (or vice versa).

In use, the jig support is external to the limb, with the internal surface of the jig support facing the limb.

By providing a connector portion that curves/bends in a plane that is perpendicular to and transects the elongate axis of the jig support/elongate shaft, the connector portion extends laterally from the jig support/elongate shaft leaving the space between the jig support and elongate shaft clear to receive the bone.

In the first reference example, the connector portion may comprise a connector body, first socket and fixing element as described for the first aspect.

The connector portion may be connected to a head of the jig support. In the first aspect, the head of the jig support is substantially aligned with the head portion of the elongate shaft.

The connector body may further comprise a second socket proximal the jig support, the second socket for receiving the head of the jig support. The second socket may span the internal and external surfaces of the head of the jig support.

The connector portion i.e. the connector body may be a single-piece component. The connector portion i.e. the connector body may be substantially C-shaped. The first and second sockets (which may also be C-shaped) are provided at opposing end of the connector body.

In some embodiments of the first aspect or the first reference example, the jig support comprises an array of jig bores extending between the internal and external surfaces. The jig bores may be configured to provide unimpeded passage of the respective fasteners through the jig bores when guiding the fasteners through the bone. That is, the jig bores may be configured not to engage with the fasteners when guiding them through the bone. For example, each jig bore may be an unthreaded bore, having a substantially smooth, unthreaded inner surface, such that the jig bore does not engage with a threaded fastener guided therethrough. Additionally, or alternatively, the maximum diameter of each jig bore may be larger than the maximum diameter of each plate bore and/or of each fastener. Each jig bore may be cylindrical.

In the first aspect, the jig bores are each axially aligned with a respective one of the array of plate bores. The axes of the jig/plate bores may be substantially perpendicular to the axis of the elongate plate or may be angularly off-set to accommodate irregular bone profiles.

The jig support of the first aspect or the first reference example may also comprise an array of transverse bores on a side surface of the jig support, each transverse bore having an axis perpendicular to the axes of the jig bores and each transverse bore extending from the side surface into a respective one of the jig bores. The purpose of these transverse bores is to secure drill guides within the jig support as will be described in detail below.

In some embodiments of the first aspect or the first reference example, the jig support comprises a bone compression device at an opposite axial end of the jig support to the head/connector portion. The bone compression device comprises a compression bore that is slidable towards the head/connector portion. The compression bore has an axis substantially parallel to the axes of the jig bores and is configured to receive a compression screw. The use of the compression screw will be described later.

The compression bore is provided in a compression piece that is slidably mounted within the jig support in a direction aligned with the elongate axis of the jig support. The internal surface of the jig support comprises a slot aligned with the compression piece and exposing the compression bore.

The jig support may further comprise an actuator for effecting sliding of the compression piece within the jig support (and movement of the compression bore/compression screw within the slot on the internal surface of the jig support). The actuator may be, for example, a threaded screw mounted in an adjustment bore at the axial end (opposing the head) of the jig support. The adjustment bore will have an axis parallel with the elongate axis of the jig support. The extension of the threaded screw from the adjustment bore within the jig support can be adjusted by rotation to effect the axial positioning of the compression piece within the jig support and the positioning of the compression bore/compression screw within the slot.

The assembly of the first aspect is used in combination with a number of additional components (e.g. the insertion tool, alignment screw, connector fixing fastener and compression screw previously described) in methods described below to affix the orthopaedic plate to a fractured bone. As described above, the assembly of the first aspect, the orthopaedic plate of the second aspect and the jig of the first reference example can be used in method of transosseous plating where the plate is inserted against a deep surface of the bone with the bone interposed between the plate and the superficial surface of the bone.

Accordingly, in a second reference example, there is provided an orthopaedic method for securing an orthopaedic plate to a bone within a patient's limb, the plate having an elongate shaft with a bone contact surface and an array of plate bores, the method comprising:
inserting the plate with the elongate shaft against a deep or surgically inaccessible surface of the bone; and
fixing the elongate shaft to the bone by passing a tip portion of a fastener through the bone from a surgically accessible or superficial surface of the bone to engage with a respective one of the plate bores.

The surgically accessible or superficial surface of the bone is generally radially outwards of the surgically inaccessible or deep surface of the bone within the limb proximal the patent's skin.

The plate may be inserted through a percutaneous incision which may be distant from the fracture or bone surgical site. The plate is advanced along the bone with an insertion portion of the plate leading.

After positioning the plate with the bone contact surface against the surgically inaccessible or deep surface of the bone, a head portion of the plate (axially opposed to the insertion portion) may be affixed to the bone. Head fixing fasteners are advanced through head bores in the head portion of the plate from an opposing surface to the bone contact surface and into the deep surface of the bone. This may be done via an appropriate surgical incision.

The method may further comprise (prior to fixing the bone to the elongate shaft) pushing the bone towards the plate by providing a pushing force on the bone (e.g. on the superficial surface of the bone) in a direction perpendicular to the elongate axis of the elongate shaft.

The method may further comprise (prior to fixing the bone to the elongate shaft) pulling the plate towards the bone (i.e. towards the deep surface of the bone) by providing a pulling force on the plate in a direction perpendicular to the elongate axis of the elongate shaft.

It will be clear that the pushing and pull forces described above will be in opposing directions.

The method may comprise providing a jig having a jig support rigidly connected to the plate. The jig support may be connected to the plate prior to insertion of the against the bone. As the plate is inserted to position the elongate shaft against a deep surface of the bone, the jig support is positioned proximal the surgically accessible or superficial surface of the bone. In this way, the bone is received in a longitudinally-extending space between the elongate shaft and the jig support.

The pushing force referred to above may be applied by providing an array of jig bores in the jig support and inserting at least one pushing element (which may be a bone pusher or may be a drill guide as discussed below) into a respective jig bore to press against the superficial surface of the bone. The extension of the pushing element(s) from the jig bores into the bone-receiving space may be locked by locking fasteners extending through transverse bores in the jig support, each transverse bore extending transversely though the jig support to a respective one of the jig bores to contact a pushing element (e.g. a bone pusher or drill guide) to lock it in place and prevent its movement.

The pulling force may be applied by providing (as part of the array of plate bores) a conical pulling plate bore having a threaded conical portion tapering from a maximum diameter proximal the bone contact surface to a minimum diameter distal the bone contact surface. A pulling screw having a parallel thread matching the minimum diameter of the conical portion may be advanced through the respective jig bore, through the bone from the superficial surface and into the threaded conical pulling plate bore where the intermeshing of the conical and cylindrical bores will pull the plate towards the jig (and thus towards the bone interposed between the plate and jig support).

Where the bone fracture requires compression, the method may comprise (prior to fixing the plate to the bone) applying an axial force to the bone in a direction towards the head portion of the plate. This axial force may be applied by a compression screw inserted into the bone (from the superficial bone surface) transverse to the axis of the bone, on an opposing axial side of the fracture to the head portion of the plate, the compression screw being axially slidable in a direction towards the head portion of the plate (which is fixed to the head of the bone). This axial force helps reduce the fracture and align the fractured portions of bone.

Once the fracture or surgical site has been compressed if required using the compression screw and adjustment screw above, the method may comprise further compression of the surgical site by drilling a stabilisation hole through the bone from the surgically accessible bone surface to the deep bone surface, providing a conical stabilisation plate bore (as part of the array of plate bores) having a threaded conical portion tapering from a maximum diameter proximal the bone contact surface to a minimum diameter distal the bone contact surface and passing a stabilisation screw having a parallel thread matching the minimum diameter of the threaded conical portion through the stabilisation hole in the bone from the superficial surface and into the conical stabilisation plate bore where the intermeshing of the conical and cylindrical bores will pull the plate towards the bone and stabilise the compressed fracture. The stabilisation hole through the bone may be provided across the fracture or through a portion of the bone away from the fracture axially opposite the head portion.

The method comprises providing a cylindrical insertion portion bore (as part of the array of plate bores) and passing an alignment screw through the respective jig bore, through the bone from the superficial surface and into the cylindrical insertion portion bore. This ensures that the elongate shaft of the plate and the jig support remain in alignment.

The step of fixing the plate to the bone by passing the tip portion of the fastener through the bone may comprise drilling a fixing hole through the bone from the surgically accessible or superficial surface to the deep or surgically inaccessible surface.

A plurality of fasteners is preferably used to fix the plate to the bone and thus a plurality of fixing holes will be drilled.

For drilling the fixing hole(s) and/or the stabilisation hole, the method may comprise providing a respective drill guide extending from the jig support (through a respective jig bore) to the surgically accessible or superficial surface of the bone. The drill guide(s) may additionally be used as described above to push on the surgically accessible or superficial surface of the bone to force the bone towards the bone contact surface of the plate. The drill guide(s) are locked in place i.e. their extension from the jig support into the bone-receiving space between the jig support and the plate is fixed using locking fasteners as described above.

The/each drill guide is hollow and defines a drill path for a drill bit from the jig to the bone. The drill bit is kept on the correct drill path (axially aligned with the respective plate bore) as it then advances through the bone to the deep surface of the bone and the bone contact surface of the elongate plate.

The method may comprise controlling the extension of the drill bit from the deep surface of the bone into the plate hole. This may be effected by using a drill stop associated with the drill guide for adjusting the axial length of the drill path (by abutting against the drill and preventing further advancement of the drill bit) and precluding extension into the plate hole beyond a predetermined amount. The extension of the drill bit into the plate hole may be accommodated in a cylindrical portion of the plate hole proximal the bone contact surface of the plate.

The step of fixing the plate to the bone by advancing the tip portion of the fastener(s) through the bone may comprise passing the fastener(s) through the respective jig bore provided in the jig support prior to passing the tip portion(s) through the fixing hole(s) in the bone from the superficial surface.

The method may comprise locking the tip portion of the/each fastener in the respective plate bore. This may be achieved by providing (as part of the array of plate bored) a conical plate bore having a threaded conical portion tapering from a maximum diameter proximal the bone contact surface to a minimum diameter distal the bone contact surface. Locking can be effected by using a fastener having a conical tip portion with a thread that matches the thread of the conical portion of the plate bore. Further details of a suitable fastener are described below.

Other methods of locking the tip portion(s) of the fastener(s) in the respective plate bore(s) are also envisaged such as using adhesive.

After fixing the plate to the bone, jig support may be disconnected from the plate.

The method of the second reference example may be effected using the assembly of the first aspect.

In a third reference example not falling within the scope of the invention, there is provided an orthopaedic method for securing a plate to a bone using the assembly according to the first aspect, the method comprising:
inserting the plate to position the bone within the longitudinally-extending bone-receiving space between the elongate shaft and the jig support with the bone contact surface of the elongate shaft against the surface of the bone; and
passing the tip portion of a fastener through the bone to engage with a respective one of the plate bores.

In order to position the bone between the plate and jig support and to pass the tip portion of the fastener through the bone (i.e. to effect transosseous plating), the plate will be positioned with the bone contact surface against the surgically inaccessible or deep surface of the bone and the tip portion of the fastener will be passed through the bone from the surgically accessible or superficial surface. The jig support will be provided proximal the surgically accessible or superficial surface of the bone.

The surgically accessible or superficial surface of the bone is generally radially outwards of the surgically inaccessible or deep surface of the bone within the limb proximal the patent's skin.

The method may comprise a first step of connecting the jig to the plate using the connector portion. The locator tabs on the fixing element may be seated in locator indents on the head portion and a connector fixing fastener may be passed through the fixing bore into one of the head bores for fixing the fixing element to the head portion of the plate.

The plate may be inserted through a percutaneous incision which may be distant from the fracture or surgical site. The plate is advanced along the bone with the insertion portion i.e. the insertion axial end of the plate leading. The rounded insertion axial end and/or tapered insertion portion provides for a clean passage of the plate through tissue adjacent the bone.

An insertion tool may be used to push the plate through the tissue. The method may comprise engaging the insertion tool with the tool receiving portion on the head portion of plate. For example, the method may comprise inserting a tool portion into a recess provided on the head portion of the plate.

As the plate is inserted to position the elongate shaft against a deep surface of the bone (e.g. by pushing using the insertion tool), the jig support is positioned proximal the surgically accessible or surface of the bone. In this way, the bone is received in a longitudinally-extending space between the elongate shaft and the jig support.

After positioning the plate with the bone contact surface against the deep surface of the bone, the head portion of the plate (axially opposed to the insertion portion) may be affixed to the bone. Head fixing fasteners are advanced through the head bores in the head portion of the plate from the opposing surface to the bone contact surface and into the deep surface of the bone.

The method may further comprise (prior to fixing the bone to the elongate shaft) pushing the bone towards the plate by providing a pushing force on the bone (e.g. on the surgically accessible or superficial surface of the bone) in a direction perpendicular to the elongate axis of the elongate shaft.

The pushing force may be applied by inserting at least one pushing element into a respective jig bore to press against the superficial surface of the bone. The extension of the pushing element(s) from the jig bores into the bone-receiving space may be locked by locking fasteners extending through the transverse bores in the jig support, each transverse bore extending transversely though the jig support to a respective one of the jig bores to contact a pushing element to lock it in place and prevent its movement.

For example, the method may comprise inserting (and locking) a bone pusher in one of the jig bores e.g. into a jig bore distal the head of the jig support. The bone pusher may be a solid element and may have a pointed end for abutment with the superficial surface of the bone.

The pushing force may additionally or alternatively be provided by inserting (and locking) at least one drill guide extending from the jig support to abut against the superficial surface of the bone. The drill guide may be a hollow member providing a drill path for a drill bit to the bone contact surface of the elongate plate.

The method may further comprise (prior to fixing the bone to the elongate shaft) pulling the plate towards the bone by providing a pulling force on the plate in a direction perpendicular to the elongate axis of the elongate shaft.

The pulling force may be applied by providing (as part of the array of plate bores) a conical pulling plate bore having a threaded conical portion tapering from a maximum diameter proximal the bone contact surface to a minimum diameter distal the bone contact surface. A pulling screw having a parallel thread matching the minimum diameter of the threaded conical portion may be advanced through the respective jig bore, through the bone from the surgically accessible or superficial surface and into the conical pulling plate bore where the intermeshing of the conical and cylindrical bores will pull the plate towards the jig (and thus towards the bone interposed between the plate and jig support).

Where the bone fracture requires compression, the method may comprise (prior to fixing the plate to the bone) applying an axial force to the bone in a direction towards the head portion of the plate.

This axial force may be applied using the bone compression device described above for the first aspect and the first reference example. The method may comprise passing the compression screw through the compression bore within the compression piece, through the slot in the internal surface of the jig support and into the bone from the surgical accessible or superficial surface transverse to the axis of the bone, on an opposing axial side of the fracture to the head portion of the plate.

The compression device actuator may be used to effect sliding of the compression piece within the jig support towards the head of the jig support. Thus, as the compression piece is moved towards the jig support head, the compression bore and compression screw move within the slot towards the head of the jig support and an axial force (towards the head of the jig support) is applied to the bone via the sliding compression screw. This will reduce and compress the fracture or surgical site in the bone.

Once the fracture or surgical site has been compressed, the method may comprise drilling a stabilisation hole through the bone from the surgically accessible or superficial bone surface to the deep bone surface, providing a conical stabilisation plate bore (as part of the array of plate bores) having a threaded conical portion tapering from a maximum diameter proximal the bone contact surface to a minimum diameter distal the bone contact surface and passing a stabilisation screw having a parallel thread matching the minimum diameter of the threaded conical portion through the stabilisation hole in the bone from the superficial surface and into the conical stabilisation plate bore where the intermeshing of the conical and cylindrical bores will pull the plate towards the bone and stabilise the compressed fracture. The stabilisation hole through the bone may be provided across the fracture or through a portion of the bone away from the fracture axially opposite the head portion.

The method may comprise providing a cylindrical insertion portion plate bore (as part of the array of plate bores) and passing an alignment screw through the respective jig bore through the bone from the superficial surface and into the cylindrical alignment plate bore. This ensures that the elongate shaft of the plate and the jig support remain in alignment.

The step of fixing the plate to the bone by passing the tip portion of the fastener through the bone may comprise drilling a fixing hole through the bone from the superficial surface to the deep surface.

A plurality of fasteners is preferably used to fix the plate to the bone and thus a plurality of fixing holes will be drilled.

For drilling the fixing hole(s) and/or the stabilisation hole, the method may comprise providing a respective drill guide extending from the jig support (through a respective jig bore) to the superficial surface of the bone. The drill guide(s) may additionally be used as described above to push on the superficial surface of the bone to force the bone towards the bone contact surface of the plate. The drill guide(s) are locked in place i.e. their extension from the jig support into the bone-receiving space between the jig support and the plate is fixed using locking fasteners as described above.

The/each drill guide is hollow and defines a drill path for a drill bit from the jig to the bone. The drill bit is kept on the correct drill path (axially aligned with the respective plate bore) as it then advances through the bone to the deep surface of the bone and the bone contact surface of the elongate plate.

The method may comprise controlling the extension of the drill bit from the deep surface of the bone into the plate hole. This may be effected by using a drill stop associated with the drill guide for adjusting the axial length of the drill path (by abutting against the drill and preventing further advancement of the drill bit) and precluding extension into the plate hole beyond a predetermined amount. The extension of the drill bit into the plate hole may be accommodated in a cylindrical portion of the plate hole proximal the bone contact surface of the plate.

The step of fixing the plate to the bone by advancing the tip portion of the fastener(s) through the bone may comprise passing the fastener(s) through the respective jig bore provided in the jig support prior to passing the tip portion(s) through the fixing hole(s) in the bone from the superficial surface.

The method may comprise locking the tip portion of the/each fastener in the respective plate bore. This may be achieved by providing (as part of the array of plate bored) a conical plate bore having a threaded conical portion tapering from a maximum diameter proximal the bone contact surface to a minimum diameter distal the bone contact surface. Locking can be effected by using a fastener having a conical tip portion with a thread that matches the thread of the conical portion of the plate bore. Further details of a suitable fastener are described below.

Other methods of locking the tip portion(s) of the fastener(s) in the respective plate bore(s) are also envisaged such as using adhesive.

After fixing the plate to the bone, jig support may be disconnected from the plate.

In order to effect the method of the third reference example discussed above, there is provided a kit for securing an orthopaedic plate to a bone, the kit comprising an assembly according to the first aspect and further comprising one or more of the following:
a) a connector fixing fastener for connecting the connector portion of the jig to the plate;
b) an insertion tool for pushing the plate through the tissue along the length of the bone;
c) at least one head fixing fastener for affixing the head portion of the plate to the bone;
d) at least one pushing element such as a bone pusher which may be a solid element and may have a pointed end for abutment with the superficial surface of the bone to push a bone in the bone-receiving space towards the bone contact surface of the elongate plate;
e) a pulling screw having a cylindrical thread matching the minimum diameter of the conical plate bore for extending through a bone in the bone-receiving space into one of the array of plate bores for pulling the elongate shaft towards the bone;
f) a compression screw for extending through the compression bore in the compression device into a bone within the bone-receiving space;
g) a stabilisation screw having a parallel thread matching the minimum diameter of the threaded conical portion of the stabilisation plate bore
h) an alignment screw for extending through a jig bore distal the connector portion and through a bone in the bone-receiving space into the insertion portion bore to ensure alignment of the elongate plate and jig support;
i) at least one fastener having a tip portion for extending through a bone in the bone-receiving space and into one of the array of plate bores for fastening the plate to bone;
j) at least one drill guide for extending through a jig bore to contact a bone within the bone-receiving space, the drill guide for providing a drill path for a drill bit;
k) at least one locking fastener for locking a respective drill bit in the jig support;
l) at least one drill stop for adjusting the axial length of the drill path;
m) at least one drill bit, the/each drill bit having a needle tip.

The or each fastener for fastening the plate to bone preferably has a conical tapered threaded tip portion which matches the threaded conical portion of the respective plate bore (e.g. has the same pitch and/or lead as the respective plate bore). For example, the tip portion may comprise a conical tapered thread having more than one (e.g. two) starts.

The fastener may also have a threaded head portion at an opposing axial end to the tip portion. The thread on the head portion may have a greater pitch than the thread on the tip portion. The head portion may have a greater diameter than the tip portion i.e. the fastener may be tapered along its length from the head portion to the tip portion. The head portion may have a recess in its axial end face for receiving a locking tool for rotating the fastener to advance it through the bone and into engagement with the plate.

The radial spacing between the thread root and thread crest may be greater at the head portion than at the tip portion. The thread crests at the head portion may be sharper than the crests at the tip portion.

The fastener shank (interposed between the tip and head) may also be threaded. The pitch and lead of the shank threads may substantially match the head portion threads. The crests of the shank threads may be less sharp than the head portion threads. When the plate is affixed (fastened) to the bone by the or each fastener, the tip portion of the or each fastener is engaged with a respective plate bore, and the head portion of the or each fastener is engaged directly with a portion of the bone.

The disclosure includes the combination of the aspects and preferred features described except where such a combination is clearly impermissible or expressly avoided.

### Summary of the Figures

Embodiments will now be discussed with reference to the accompanying figures in which:
Figure 1 shows a perspective view of the assembly with an attached insertion tool and a bone *in situ*;
Figure 2 shows an enlarged view of a conical threaded plate bore;
Figure 3A shows a sectional view of the assembly with an attached insertion tool and a bone *in situ*;
Figure 3B shows an enlarged view of the engagement between the plate and insertion tool;

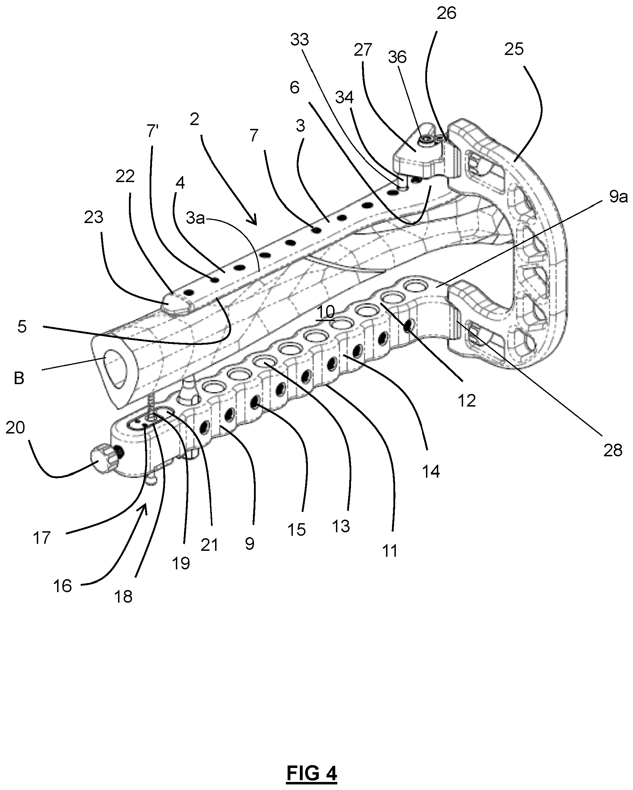

Figure 4 shows a perspective view of the assembly with a bone *in situ*;
Figure 5 shows a sectional view of the insertion end of the assembly showing the compression device;
Figure 6a shows a sectional view of the assembly showing inserted drill guides and Figure 6b shows a perspective view of a drill guide;
Figure 7A shows the drilled stabilisation hole across the fracture;
Figure 7B shows a stabilisation screw;
Figure 8 shows the insertion of the stabilisation screw across the fracture;
Figures 9 and 10 show an alignment screw in position within the assembly;
Figure 11 shows the drilling of a fixing hole using a drill bit;
Figure 12 shows a locking fastener;
Figure 13 shows a plurality of locking fasteners in situ within the assembly;
Figure 14 shows the plate affixed to the bone after removed of the jig; and
Figure 15 shows a transverse cross section through the lower leg.

### Detailed Description

Aspects and embodiments will now be discussed with reference to the accompanying figures. Further aspects and embodiments will be apparent to those skilled in the art.

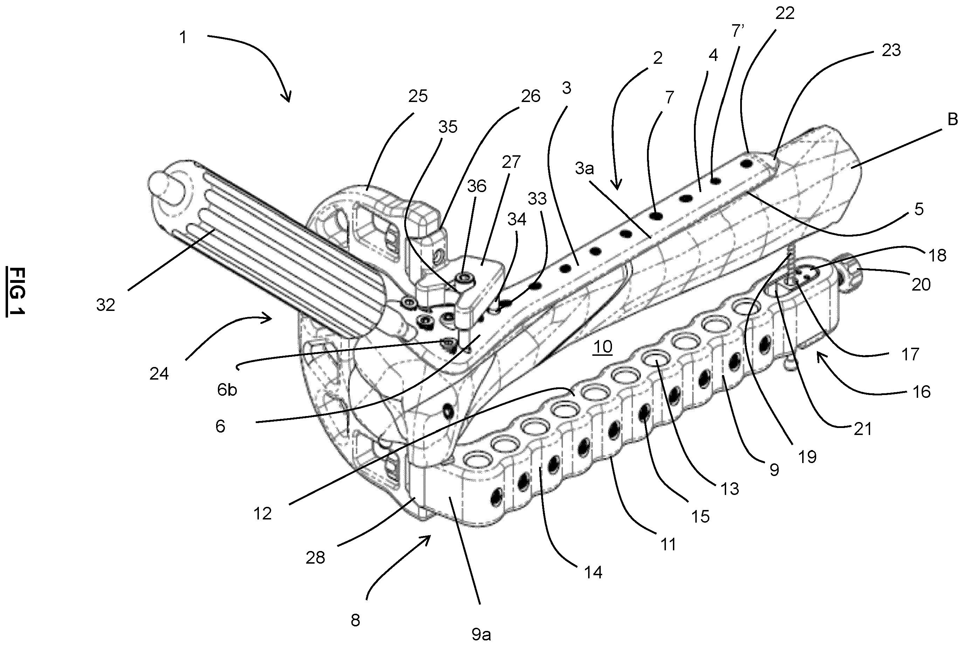

Figure 1 shows an assembly 1 for securing an orthopaedic plate 2 to a bone B which, in this embodiment is a tibia in a patent's leg. The assembly 1 comprises the orthopaedic plate 2 having an elongate shaft 3 with bone contact surface 5 configured to contact the deep surface of the bone B and an opposing surface 4. The elongate shaft 3 comprises an array of plate bores 7 which form a row of equally spaced bores aligned with the central axis of the elongate shaft 3.

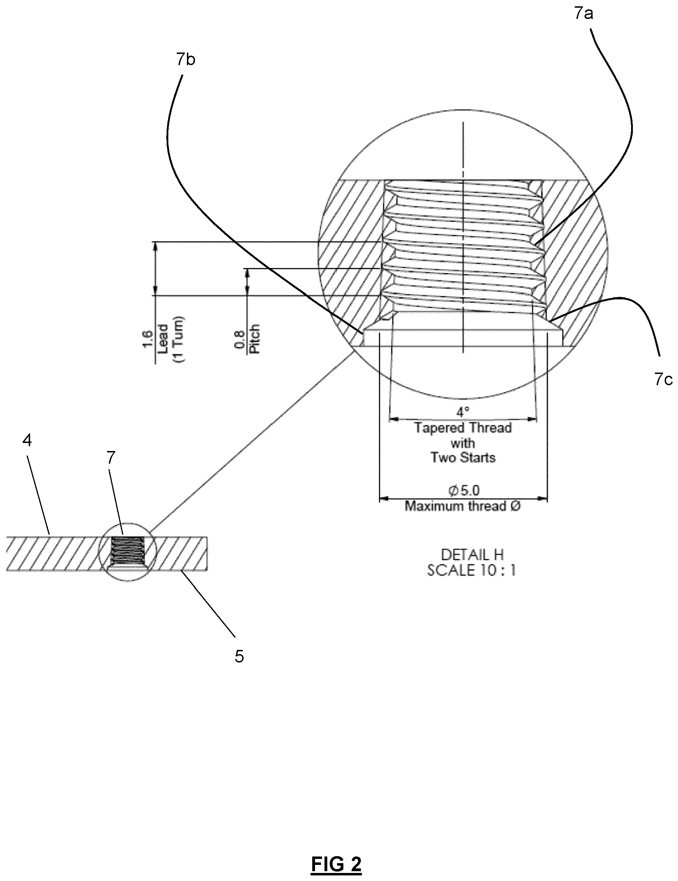

A plate bore 7 is shown in Figure 2. The plate bore 7 has a threaded portion 7a which is tapered from a maximum diameter of 5mm proximal the bone contacting surface 5 to a minimum diameter of 4mm proximal the opposing surface 4. The tapered threaded portion 7a has a pitch of 0.8mm and has a double start providing a lead of 1.6mm.

The plate bore 7 has a maximum diameter, unthreaded cylindrical portion 7b at the bone contact surface 5 with a shoulder portion 7c interposed between the tapered threaded portion 7a and cylindrical portion 7b.

The assembly 1 as shown in Figure 1 further comprises a jig 8 comprising an elongate jig support 9.

The elongate shaft 3 of the plate 2 and the elongate jig support 9 are aligned substantially parallel to each other to define a longitudinally-extending bone-receiving space 10 between the bone contact surface 5 of the elongate shaft 3 and an internal surface 12 of the jig support 9.

The jig support 9 comprises an array of jig bores 13 which extend between the internal surface 12 and an opposing external surface 11.

The axes of the jig bores 13 and plate bores 7 are substantially perpendicular to the elongate axis of the jig support 9 and elongate shaft 3. Opposing jig bores 13 and plate bores 7 (i.e. opposing across the bone-receiving space 10) are axially aligned.

The jig support 9 further comprises a side surface 14 with an array of transverse bores 15. Each transverse bore 15 has an axis perpendicular to the axis of the jig bores 13 and perpendicular to the elongate axis of the jig support 9. Each transverse bore 15 extends from the side surface 14 to join a respective jig bore 13.

The jig support 9 comprises a head 9a at one axial end and a compression device 16 at the opposing axial end. The compression device comprises a compression bore 17 with an axis substantially parallel to the axes of the jig bores 13 that is slidable towards the head 9a of the jig support 9. The compression bore 17 has and is configured to receive a compression screw 19. The use of the compression screw 19 will be described later.

The compression bore 17 is provided in a compression piece 18 that is slidably mounted within the jig support 9 in a direction aligned with the elongate axis of the jig support 9. The internal surface 12 of the jig support 9 comprises a slot 21 aligned with the compression piece 18 and exposing the compression bore 17.

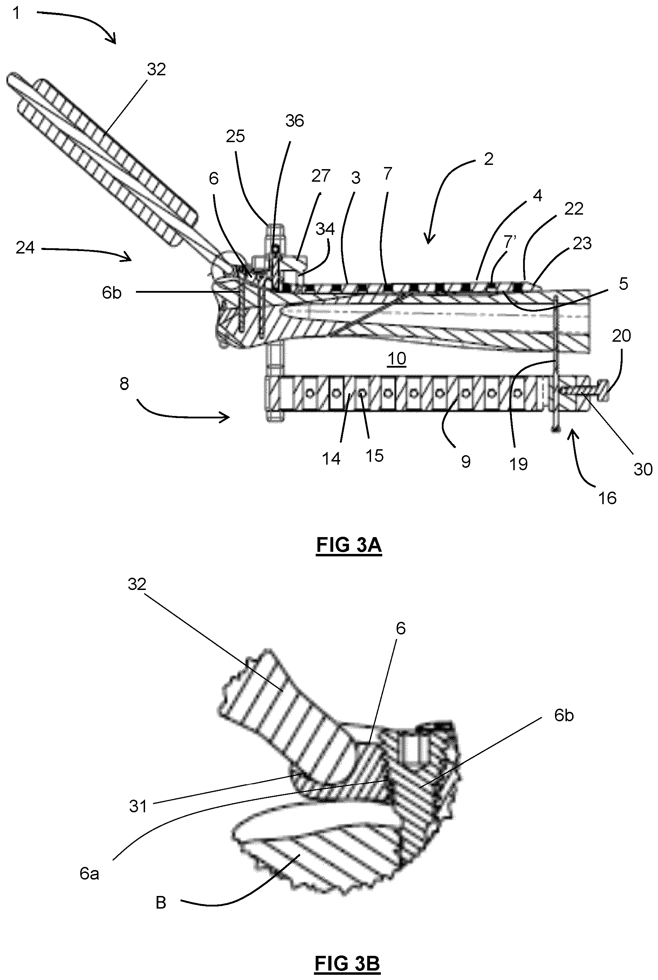

The compression device 16 further comprise an actuator 20 for effecting sliding of the compression piece 18 within the jig support 9 (and movement of the compression bore 18/compression screw 19 within the slot 21 on the internal surface 12 of the jig support 9). The actuator 20 is a threaded screw mounted in an adjustment bore 30 (see Figure 3A) at the axial end (opposing the head 9a) of the jig support 9. The adjustment bore 30 will have an axis parallel with the elongate axis of the jig support 9. The extension of the threaded screw 20 from the adjustment bore 30 within the jig support 9 can be adjusted by rotation to effect the axial positioning of the compression piece 18 within the jig support 9 and the positioning of the compression bore 18/compression screw 19 within the slot 21.

The plate 2 comprises a head portion 6 at an axial end of the elongate shaft 3. The head portion 6 has an enlarged width in a direction perpendicular to the elongate shaft 3 and perpendicular to a depth direction between the bone contact surface 5 and the opposing surface 4. The head portion 6 may comprises a plurality of threaded head bores 6a.

As is best seen in Figure 3A for example, the head portion 6 may extend out of the plane of the elongate shaft 3. The head portion 6 extends so that there is an angle of greater than 180 degrees between head portion 6 and the bone contact surface 5 and an angle of less than 180 degrees between the head portion 6 and the opposing surface 4. In this embodiment, the head portion 6 extends by an angle of around 30-40 degrees away from the plane of the elongate shaft 3 (and away from the jig support 9). This allows the elongate shaft 3 to sit against the deep surface of the bone B whilst the head portion 6 accommodates the (deep surface of the) enlarged head of the bone B.

The opposing surface 4 of the elongate shaft 3 and the head portion 6 (i.e. the surfaces configured to face away from the bone B) have a chamfered edge 3a.

The head portion 6 may comprise a tool receiving portion 31 for receiving an insertion tool 32 as shown in Figures 3A and 3B. Figure 3B is an enlarged view of the tool engagement. The tool receiving portion 31 is a recess for receiving the tool 32 and is provided in the axial end of the head portion 6. The tool 32 can be used to facilitate positioning of the plate 2 against the deep surface of the bone B as the tool 32 can be engaged in the recess 31 and used to push the elongate shaft 3 within the patient's limb so that the elongate shaft 3 lies against the bone shaft (and the head portion 6 is seated against the enlarged head of the bone B).

Insertion of the plate 2 is additionally facilitated by the provision of an insertion portion 22 on the elongate shaft 3 terminating at an insertion axial end 23 (at the opposite axial end to the head portion 6). The insertion axial end 23 is rounded in the plane of the elongate shaft 3 and the insertion portion 22 tapers along the length of the insertion portion 22 to a reduced depth (i.e. to a reduced spacing between the bone contact surface 5 and the opposing surface 4 of the elongate shaft 3). This is achieved by tapering of the opposing surface 4 towards the bone contact surface 5 of the elongate shaft 3 (and towards the jig support 9).

The array of plate bores 7 comprises a cylindrical threaded insertion portion bore 7' proximal the insertion axial end 23. The insertion portion bore 7' is configured to receive an alignment screw 85, the purpose of which will be described later.

The jig 8 may comprise a connector portion 24 extending between and rigidly connecting the jig support 9 and the orthopaedic plate 2. The connector portion 24 is provided to maintain a fixed spatial relationship between the jig support 9 and orthopaedic plate 2. The connector portion 24 is removably connected to the opposing surface 4 of the head portion 6 of the orthopaedic plate 2.

The connector portion 24 comprises a C-shaped connector body 25 that curves in a plane that is substantially perpendicular to and transects the elongate axis of the jig support 9. By providing a connector portion 24/connector body 25 that curves in a plane that is perpendicular to and transects the elongate axis of the jig support 9/elongate shaft 3, the connector portion 24 extends laterally from the jig support 9/elongate shaft 3 leaving the space 10 between the jig support 9 and elongate shaft 3 clear to receive the bone B.

The connector body 25 comprises a first socket 26 proximal the head portion 6 of the orthopaedic plate 2, the first socket 26 (which is C-shaped in the same plane as the connector body 25) houses a fixing element 27 for removably fixing the connector body 25 to the orthopaedic plate 2. The opposing surface 4 of the head portion 6 of the plate 2 comprises locator indents 33 for receiving locator tabs 34 provided on the fixing element 27. The fixing element 27 comprises a fixing bore 35 for receiving a connector fixing fastener 36 for removably fixing the fixing element 27 in one of the head bores 6a.

The connector body 25 is connected to the plate 2 prior to insertion using the jig assembly 1 for the insertion of the plate 2.

The connector body 25 further comprise a second socket 28 proximal the jig support 9. The second socket 28 has a C-shape and spans the internal and external surfaces 11, 12 of the head 9a of the jig support 9.

The jig assembly 1 is used in methods described below to affix the orthopaedic plate 2 to a fractured bone B.

By providing an assembly 1 where the orthopaedic plate 2 and jig 8 define, between them, an elongated (longitudinally-extending) space 10 for receiving a bone B (e.g. a tibia) requiring fixing, the plate 2 can be inserted into the limb (e.g. leg) through a small incision with the elongate shaft 3 positioned against the deep surface of the bone B whilst the elongate jig support 9 remains external to the limb proximal the superficial surface of the bone B.

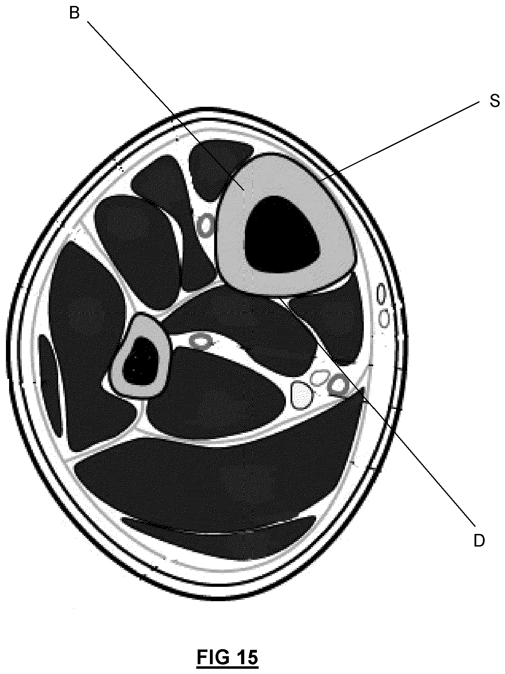

Figure 15 shows a transverse cross section through the lower leg. The surgically accessible or superficial surface S of the tibia B is adjacent the patient's skin whereas the surgically inaccessible or deep surface D is radially inwards.

The bone-receiving space 10 between the bone contact surface 5 and the jig support 9 is an unobstructed space prior to insertion into the patient's limb i.e. there are no obstructions extending transversely across the space 10 between the bone contact surface 5 and the jig support 9 so that the bone B can positioned within the space 10 without meeting impedance from any part of the assembly 1.

First, the jig 8 is connected to the plate 2 using the connector portion 24. The locator tabs 34 on the fixing element 27 are seated in locator indents 33 on the head portion 6 of the plate 2 and a connector fixing fastener 36 is passed through the fixing bore 35 into one of the head bores 6a to releasably secure the fixing element 27 to the head portion 6 of the plate 2. The connection between the fixing element 27 and the head portion 6 of the plate 2 coupled with the rigidity of the connector portion 24 ensures that the spatial relationship between the elongate shaft 3 and the jig support 9 is maintained.

The plate 2 is inserted through a percutaneous incision which may be distant from the fracture or surgical site F. The plate 2 is advanced along the bone B with the insertion portion 22 i.e. the insertion axial end 23 of the plate 2 leading. The rounded insertion axial end 23 and tapered insertion portion 22 provides for a clean passage of the plate 2 through tissue adjacent the bone B.

The insertion tool 32 is used to push the plate 2 through the tissue. As shown in Figures 1, 3A and 3B, the insertion tool 32 is engaged with the tool receiving recess 31 on the head portion 6 of the plate 2.

As the plate 2 is inserted to position (by pushing using the insertion tool 32 until the elongate shaft 3 is against the deep surface D of the bone B), the jig support 9 is positioned proximal the surgically accessible or superficial surface S of the bone B. In this way, the bone B is received in a longitudinally-extending space 10 between the elongate shaft 3 and the jig support 9 as shown in figures 1 and 3A.

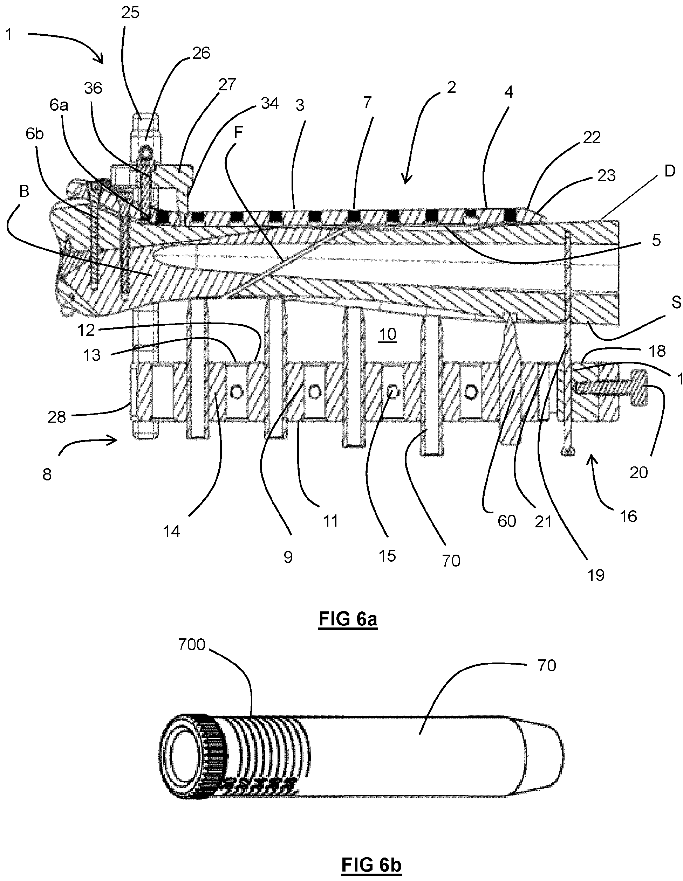

After positioning the plate 2 with the bone contact surface 5 against the surgically inaccessible or deep surface D of the bone B, the head portion 6 of the plate 2 (axially opposed to the insertion portion 22) is affixed to the bone B. Head fixing fasteners 6b are advanced through the head bores 6a in the head portion 6 of the plate 2 from the opposing surface 4 to the bone contact surface 5 and into the deep surface D of the bone B as can be seen in Figures 3A and 6, for example.

Prior to fixing the bone B to the elongate shaft 3, the bone B is pushed towards the plate 2 by providing a pushing force on the superficial surface S of the bone B in a direction perpendicular to the elongate axis of the elongate shaft 3.

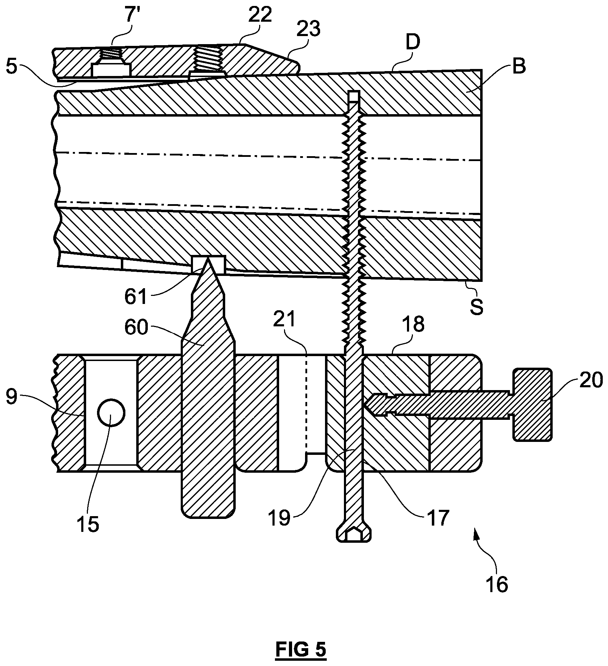

The pushing force is applied by inserting a bone pusher 60 into one of the jig bores 13 distal the head 9a to press against the superficial surface S of the bone B and force it towards the elongate shaft 3. The bone pusher 60 is a solid element with a pointed end 61 for abutment with the superficial surface S of the bone B as shown in Figure 5.

The bone pusher 60 may be locked in position be inserting a locking fastener (not shown) into the transverse bore 15 associated with the jig bore 13 housing the bone pusher 60 to limit movement of the bone pusher 60 within the jig bore 13.

The pushing force is additionally provided by inserting and locking hollow drill guides 70 extending from the jig support 9 (i.e. extending through a jig bores 13 into the bone-receiving space 10) to abut against the superficial surface S of the bone B as shown in Figure 6a. These drill guides 70 can be locked in their respective jig bores 13 using locking fasteners (not shown) in the same way as the bone pusher 60. An exemplary drill guide is shown in Figure 6b. The drill guide 70 has measuring rings 700 to accurately indicate the correct length of the fastener 100.

It can be seen in Figure 6a that the bone fracture F requires compression. An axial force is applied to the bone in a direction towards the head portion 6 of the plate 2 using the bone compression device 16 described. The compression screw 19 is passed through the compression bore 17 within the compression piece 18, through the slot 21 in the internal surface 12 of the jig support 9 and into the bone B from the superficial surface S transverse to the axis of the bone B, on an opposing axial side of the fracture F to the head portion 6 of the plate 2.

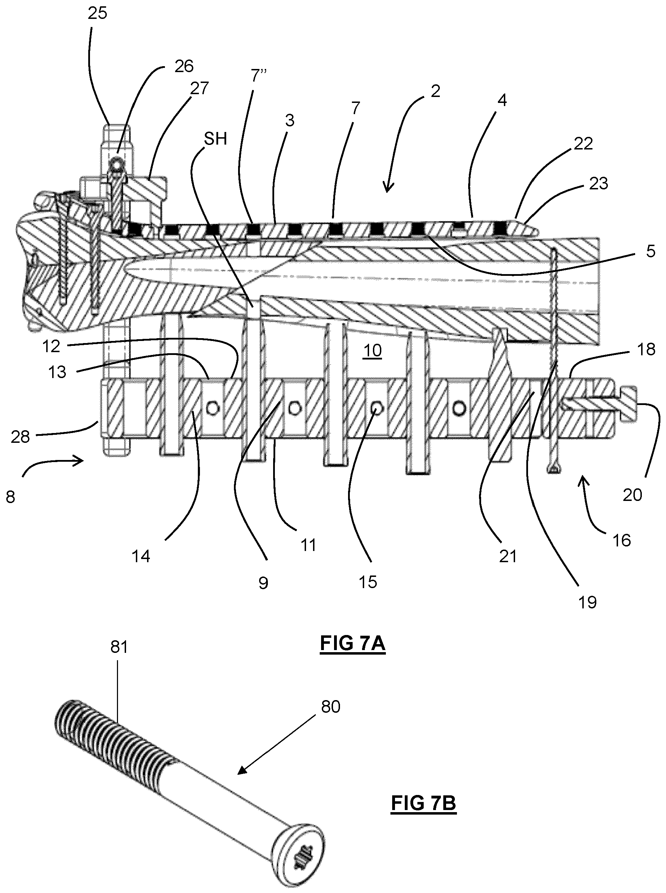

The compression device actuator 20 is used to effect sliding of the compression piece 18 within the jig support 9 towards the head 9a of the jig support 9. Thus, as the compression piece 18 is moved towards the jig support head 9a, the compression bore 17 and compression screw 19 move within the slot 21 towards the head 9a of the jig support 9 and an axial force (towards the head 9 of the jig support 9a) is applied to the bone B via the sliding compression screw 19. This reduces and compresses the fracture F in the bone B as shown in Figure 7A.

Once the fracture has been compressed, a stabilisation hole SH may be drilled through the bone B across the fracture F from the superficial bone surface S to the deep bone surface D as shown in Figure 7A. A stabilisation screw 80 such as that seen in Figure 7B is advanced through the stabilisation hole SH in the bone B from the superficial surface S and into a conical stabilisation plate bore 7" having a threaded conical portion tapering from a maximum diameter proximal the bone contact surface 5 to a minimum diameter distal the bone contact surface 5. The stabilisation plate bore 7" has the same dimensions as the plate bores 7.

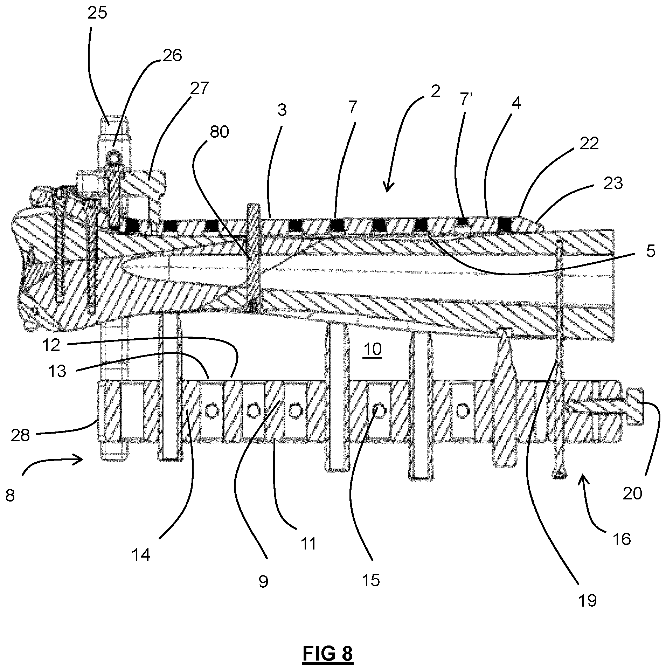

The stabilisation screw 80 has a parallel thread 81 matching the minimum diameter of the threaded conical portion of the conical stabilisation plate bore 7" and the intermeshing of the conical and cylindrical bores pulls the plate 2 towards the bone B and stabilises the compressed fracture F as shown in Figure 8.

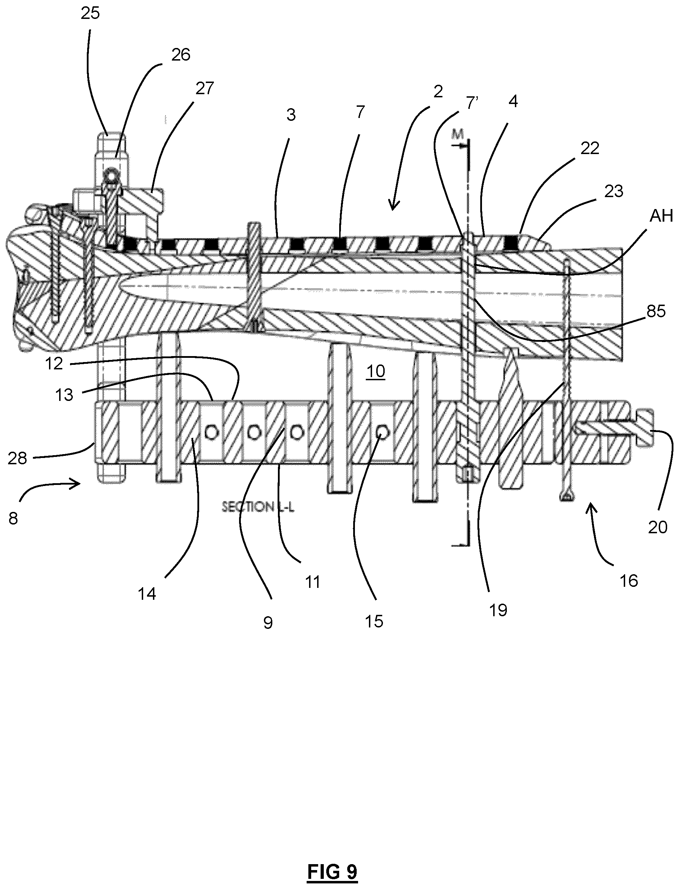

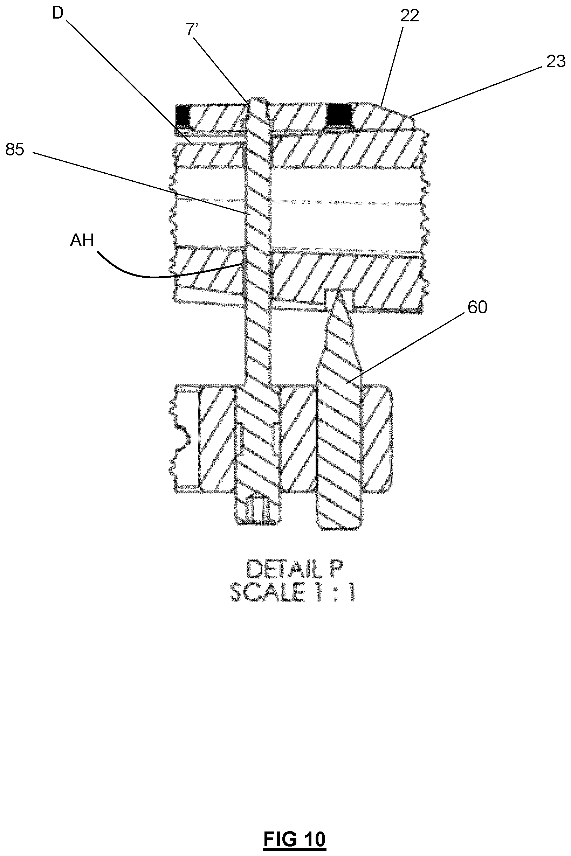

Next, an alignment screw 85 is passed through an alignment hole AH drilled through the bone B and into through the cylindrical insertion portion bore 7' as shown in Figure 9 and 10. This ensures that the elongate shaft of the plate 2 and the jig support 9 remain in alignment.

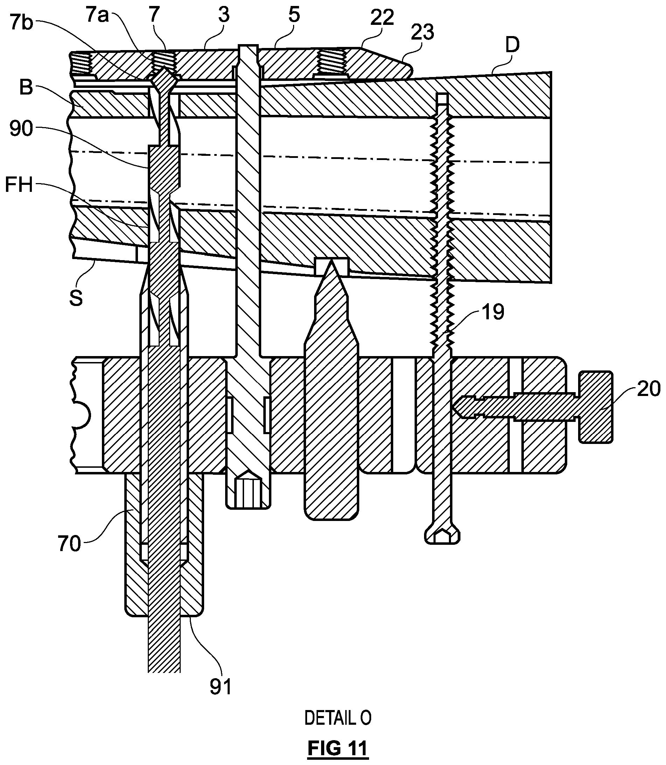

Next, a fixing hole FH is drilled through the bone B from the superficial surface S to the deep surface D as shown in Figure 11.

For drilling the fixing holes FH, the drill guides 70 (see Figure 6b) are used to defines a drill path for a drill bit 90 from the jig 8 to the bone B. The measuring rings 700 are used to indicate the correct length of the fastening, The drill bit 90 is kept on the correct drill path (axially aligned with the respective plate bore 7) by the drill guide 70 as it then advances through the bone B to the deep surface D of the bone B and the bone contact surface 5 of the elongate shaft 3.

The extension of the drill bit 90 from the deep surface D of the bone B into the plate hole 7 is controlled using a drill stop 91 which fits over the drill guide 70. The drill stop 91 abuts the external surface 11 of the jig 8 and precludes extension of the drill bit 90 into the plate hole 7 beyond a predetermined amount. The extension of the drill bit 90 into the plate hole 7 may be accommodated in the cylindrical portion 7b of the plate hole 7 proximal the bone contact surface 5 of the plate 2 to avoid any damage to the threaded portion 7a. Further extension of the drill bit 90 is prevented by the drill stop 91 abutting the external surface 11 of the jig 8.

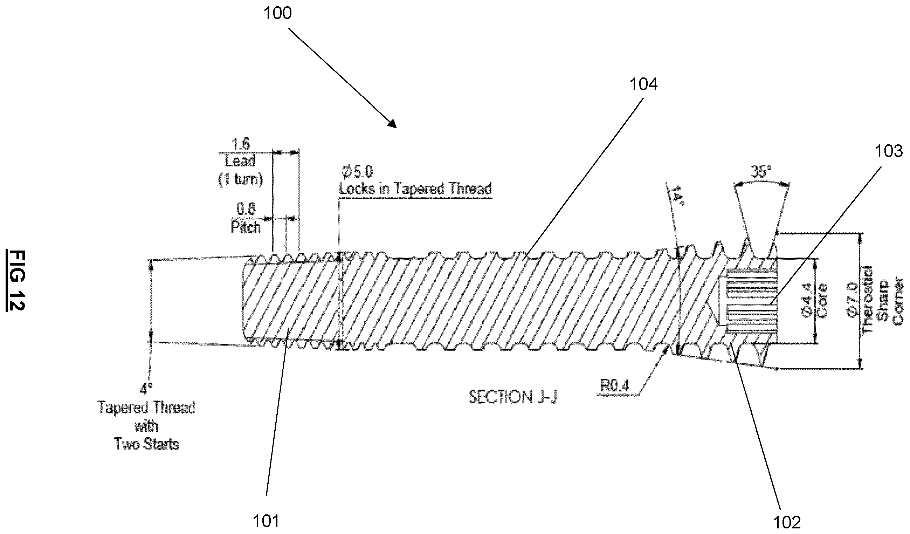

After drilling the fixing holes FH, fasteners 100 such as that shown in Figure 12 are advanced through the respective jig bore 13 provided in the jig support 9 prior to passing through the fixing hole(s) FH in the bone B from the superficial surface S.

The fastener 100 comprises a conical tapered threaded tip portion 101 which matches the threaded conical portion 7a of the respective plate bore 7 (i.e. has the same pitch (0.8mm) and lead (1.6mm) as the respective plate bore 7).

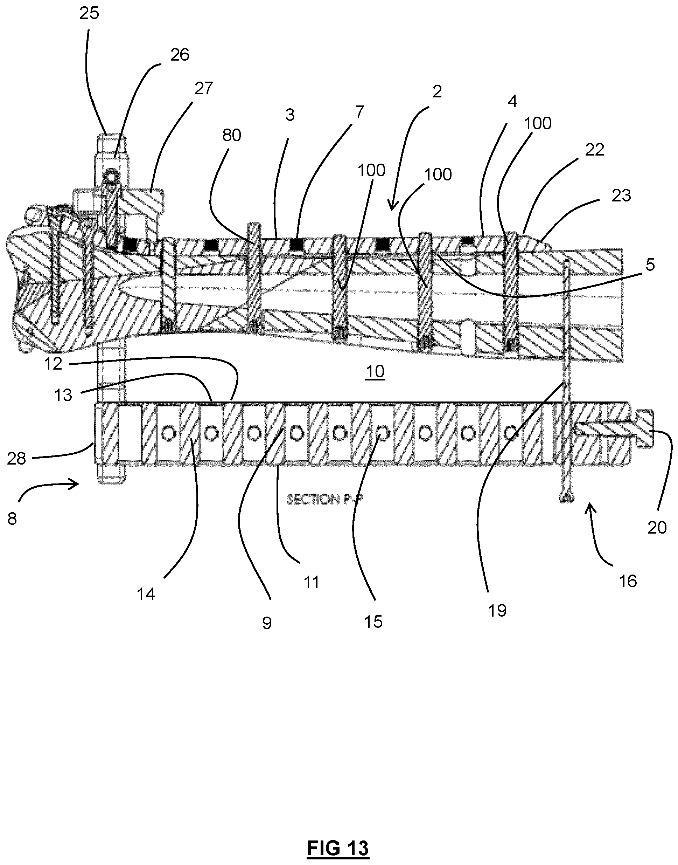

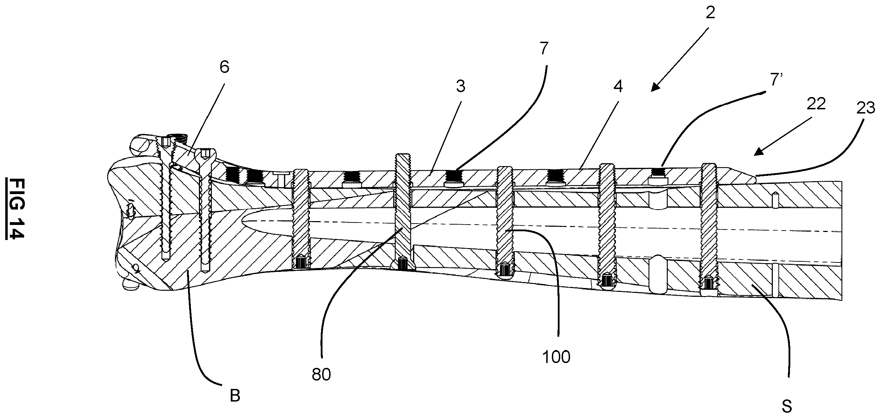

The fastener 100 also has a threaded head portion 102 at an opposing axial end to the tip portion 101. The thread on the head portion 102 has a greater pitch than the thread on the tip portion 101. This allows it to bite into the bone. Thus, the fastener is configured to affix the orthopaedic plate to the bone by engaging its tip portion 101 with a respective plate bore (e.g. by locking the tip portion in the respective plate bore) and by engaging its threaded head portion 102 directly with a portion of the bone (i.e. by biting into the bone). This is illustrated in Figures 13 and 14. The head portion has a greater diameter (7mm) than the tip portion 101 i.e. the fastener 100 is tapered along its length from the head portion 102 to the tip portion 101. The head portion 102 has a recess 103 in its axial end face for receiving a locking tool (not shown) for rotating the fastener 100 to advance it through the bone B in the fixing hole FH and into engagement with the plate 2.

The radial spacing between the thread root and thread crest is greater at the head portion 102 than at the tip portion 101. The thread crests at the head portion 102 are sharper than the crests at the tip portion 101.

The fastener shank 104 (interposed between the tip 101 and head 102) is also threaded. The pitch and lead of the shank threads may substantially match the head portion threads. The crests of the shank threads may be less sharp than the head portion threads.

As the conical tip portion 101 of the fastener 100 engages the respective plate bore 7, the intermeshing of the conical bores effects locking of the tip portion 101 of the fastener 100 in the plate hole 7.

A shown in Figure 13, a plurality of fasteners 100 are locked into a plurality of plate bores 7.

After fixing the elongate shaft 3 to the bone B, jig support 9 may be disconnected from the plate 2 by detaching the fixing element 27 from the head portion 6 of the plate 2 as shown in Figure 14.

The heads 102 of the fasteners 100 will sit at the superficial surface S of the bone B. This "transosseous plating" positions the plate 2 deep within the limb and thus does not cause skin breakdown whilst also avoiding the need for any significant surgical exposure and reducing the risk of nerve/artery damage during fastener insertion due to the absence of arteries proximal the superficial surface S of the bone B.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the disclosure in diverse forms thereof.

While the disclosure has been described in conjunction with the exemplary embodiments described above, many equivalent modifications and variations will be apparent to those skilled in the art. Accordingly, the exemplary embodiments set forth above are considered to be illustrative and not limiting. Various changes to the described embodiments may be made without departing from scope of the claims.

For the avoidance of any doubt, any theoretical explanations provided herein are provided for the purposes of improving the understanding of a reader. The inventors do not wish to be bound by any of these theoretical explanations.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. An orthopaedic plate (2) for securing to a bone, the plate (2) having an elongate shaft (3) with a bone contact surface (5) configured to contact the bone and an opposing surface (4), the elongate shaft (3) having an array of threaded bores, each bore for receiving a respective fastener (100), **characterised in that** at least one of the array of threaded bores is a conical plate bore (7) tapering from a maximum diameter at the bone contact surface (5) to a minimum diameter at the opposing surface (4).

2. An assembly (1) for securing an orthopaedic plate (2) to a bone, the assembly comprising:
an orthopaedic plate (2) according to claim 1; and
a jig (8) comprising an elongate jig support (9),
wherein
the elongate shaft (3) of the plate (2) and the elongate jig support (9) are aligned parallel to each other to define a longitudinally-extending bone-receiving space (10) between the bone contact surface (5) of the elongate shaft (3) and the jig support.

3. An assembly (1) according to claim 2 wherein the jig (8) comprises a connector portion (24) connected between the plate (2) and an axial end of the jig support (9), the connector portion (24) being curved or bent between the jig support (9) and plate (2) and lying in a plane that is substantially perpendicular to and transects the elongate axis of the jig support (9).

4. An assembly (1) according to claim 2 or 3 wherein the plate (2) comprises a head portion (6) at an axial end of the elongate shaft (3), the plate (2) comprising at least one head bore (6a), the head portion (6) extending at an angle to the elongate shaft (3) away from the jig support (9).

5. An assembly (1) according to claim 4 wherein the head portion (6) comprises a tool receiving portion (31) for receiving a tool (32).

6. An assembly (1) according to any one of the preceding claims, wherein the at least one conical plate bore (7) comprises a conical portion (7a) tapering from a maximum diameter proximal the bone contact surface to a minimum diameter distal the bone contact surface, and a cylindrical portion (7b) proximal the bone contact surface (5) with a shoulder between the cylindrical portion (7b) and the conical portion (7a).

7. An assembly (1) according to any one of the preceding claims wherein the elongate shaft (3) comprises a tapered insertion portion (22) terminating at an insertion axial end (23).

8. An assembly (1) according to claim 7 wherein the array of plate bores (7) comprises an insertion portion bore (7') at or proximal the insertion axial end (23), wherein the insertion portion bore (7') is a cylindrical threaded bore.

9. An assembly (1) according to any one of the preceding claims wherein the jig support (9) comprises an array of jig bores (13) on an internal surface facing the bone-receiving space (10), each jig bore axially aligned with a respective one of the array of plate bores (7).

10. An assembly (1) according to claim 9 wherein the jig support (9) further comprises an array of transverse bores (15) on a side surface of the jig support, each transverse bore (15) having an axis perpendicular to the axes of the jig bores (13) and each transverse bore (15) extending from the side surface into a respective one of the jig bores.

11. An assembly (1) according to any one of claims 3 to 10 wherein the jig support (9) comprises a bone compression device (16) at an opposite axial end of the jig support (9) to the connector portion (24), the bone compression device comprising a compression bore (17) that is slidable towards the connector portion (24).

12. An assembly (1) according to claim 11 wherein the compression bore (17) has an axis substantially parallel to the axes of the plate bores (7) and is provided in a compression piece (18) that is slidable within the jig support (9) in a direction aligned with the elongate axes of the plate (2) and jig support (9).

13. An assembly (1) according to claim 12 further comprising an actuator (20) for sliding the compression piece (18) within the jig (8).

14. A kit for securing an orthopaedic plate (2) to a bone, the kit comprising an assembly (1) according to any one of claims 2 to 13 further comprising one or more of the following:
a) a connector fixing fastener (36) for connecting a/the connector portion (24) of the jig (8) to the plate (2);
b) an insertion tool (32) for pushing the plate (2) through the tissue along the length of the bone;
c) at least one head fixing fastener (6b) for affixing a/the head portion (6) of the plate (2) to the bone;
d) at least one pushing element for abutment with the superficial surface of the bone to push a bone in the bone-receiving space (10) towards the bone contact surface (5) of the elongate plate;
e) a pulling screw having a cylindrical thread for pulling the elongate shaft (3) towards the bone;
f) a compression screw for extending through a/the compression bore in a/the compression device into a bone within the bone-receiving space (10);
g) a stabilisation screw having a parallel thread for stabilising a fracture;
h) an alignment screw for extending through a jig bore (13) distal the connector portion (24) and through a bone in the bone-receiving space (10) into a/the insertion portion bore (7') to ensure alignment of the elongate plate and jig support (9);
i) at least one fastener (100) having a tip portion for extending through a bone in the bone-receiving space (10) and into one of the array of plate bores (7) for fastening the plate (2) to bone;
j) at least one drill guide for extending through a jig bore (13) to contact a bone within the bone-receiving space (10), the drill guide for providing a drill path for a drill bit;
k) at least one locking fastener for locking a respective drill bit in the jig support (9);
l) at least one drill stop for adjusting the axial length of the drill path;
m) at least one drill bit, the/each drill bit having needle tip.

15. A kit according to claim 14 comprising at least one fastener (100) having a tip portion for extending through a bone in the bone-receiving space (10) and into one of the array of plate bores (7) for fastening the plate (2) to bone wherein the tip portion of the/each fastener (100) comprises a respective tapered conical thread.

## Patentansprüche

1. Orthopädische Platte (2) zur Befestigung an einem Knochen, wobei die Platte (2) einen länglichen Schaft (3) mit einer Knochenkontaktfläche (5) aufweist, die ausgelegt ist, um den Knochen zu berühren, und einer gegenüberliegenden Fläche (4), wobei der längliche Schaft (3) eine Anordnung von Gewindebohrungen aufweist, wobei jede Bohrung zum Aufnehmen eines entsprechenden Befestigungselements (100) dient, **dadurch gekennzeichnet, dass** zumindest eine aus der Anordnung von Gewindebohrungen eine konische Plattenbohrung (7) ist, die sich von einem maximalen Durchmesser an der Knochenkontaktfläche (5) bis zu einem minimalen Durchmesser an der gegenüberliegenden Fläche (4) hin verjüngt.

2. Anordnung (1) zur Befestigung einer orthopädischen Platte (2) an einem Knochen, wobei die Anordnung Folgendes umfasst:
eine orthopädische Platte (2) nach Anspruch 1; und
eine Einspannvorrichtung (8), die einen länglichen Einspannvorrichtungsträger (9) umfasst,
wobei der längliche Schaft (3) der Platte (2) und der längliche Einspannvorrichtungsträger (9) parallel zueinander ausgerichtet sind, um einen sich der Länge nach erstreckenden Knochenaufnahmeraum (10) zwischen der Knochenkontaktfläche (5) des länglichen Schafts (3) und dem Einspannvorrichtungsträger zu definieren.

3. Anordnung (1) nach Anspruch 2, wobei die Einspannvorrichtung (8) einen Verbinderabschnitt (24) umfasst, der zwischen der Platte (2) und einem axialen Ende des Einspannvorrichtungsträgers (9) angebunden ist, wobei der Verbinderabschnitt (24) zwischen dem Einspannvorrichtungsträger (9) und der Platte (2) gekrümmt oder gebogen ist und in einer Ebene liegt, die im Wesentlichen orthogonal zur Längsachse des Einspannvorrichtungsträgers (9) ist und diese schneidet.

4. Anordnung (1) nach Anspruch 2 oder 3, wobei die Platte (2) einen Kopfabschnitt (6) an einem axialen Ende des länglichen Schafts (3) umfasst, die Platte (2) zumindest eine Kopfbohrung (6a) umfasst und sich der Kopfabschnitt (6) in einem Winkel zum länglichen Schaft (3) vom Einspannvorrichtungsträger (9) weg erstreckt.

5. Anordnung (1) nach Anspruch 4, wobei der Kopfabschnitt (6) einen Werkzeugaufnahmeabschnitt (31) für die Aufnahme eines Werkzeugs (32) umfasst.

6. Anordnung (1) nach einem der vorangegangenen Ansprüche, wobei die zumindest eine konische Plattenbohrung (7) einen konischen Abschnitt (7a), der sich von einem maximalen Durchmesser proximal zur Knochenkontaktfläche zu einem minimalen Durchmesser distal zur Knochenkontaktfläche erstreckt, und einen zylindrischen Abschnitt (7b) proximal zur Knochenkontaktfläche (5) mit einer Schulter zwischen dem zylindrischen Abschnitt (7b) und dem konischen Abschnitt (7a) umfasst.

7. Anordnung (1) nach einem der vorangegangenen Ansprüche, wobei der längliche Schaft (3) einen verjüngten Einführungsabschnitt (22) umfasst, der an einem Einführungsaxialende (23) endet.

8. Anordnung (1) nach Anspruch 7, wobei die Anordnung von Plattenbohrungen (7) eine Einführungsabschnittsbohrung (7') am oder proximal zum Einführungsaxialende (23) umfasst, wobei die Einführungsabschnittbohrung (7') eine zylindrische Gewindebohrung ist.

9. Anordnung (1) nach einem der vorangegangenen Ansprüche, wobei der Einspannvorrichtungsträger (9) eine Anordnung von Einspannvorrichtungsbohrungen (13) auf einer Innenoberfläche umfasst, die dem Knochenaufnahmeraum (10) zugewandt ist, wobei jede Einspannvorrichtungsbohrung axial mit einer entsprechenden der Anordnung von Plattenbohrungen (7) ausgerichtet ist.

10. Anordnung (1) nach Anspruch 9, wobei der Einspannvorrichtungsträger (9) ferner eine Anordnung von Querbohrungen (15) auf einer Seitenoberfläche des Einspannvorrichtungsträgers umfasst, wobei jede Querbohrung (15) eine Achse aufweist, die normal auf die Achsen der Einspannvorrichtungsbohrungen (13) steht, und wobei sich jede Querbohrung (15) von der Seitenoberfläche in eine entsprechende der Einspannvorrichtungsbohrungen erstreckt.

11. Anordnung (1) nach einem der Ansprüche 3 bis 10, wobei der Einspannvorrichtungsträger (9) eine Knochenkompressionsvorrichtung (16) an einem dem Verbinderabschnitt (24) entgegengesetzten axialen Ende des Einspannvorrichtungsträgers (9) umfasst, wobei die Knochenkompressionsvorrichtung eine Kompressionsbohrung (17) umfasst, die in Richtung des Verbinderabschnitts (24) verschiebbar ist.

12. Anordnung (1) nach Anspruch 11, wobei die Kompressionsbohrung (17) eine Achse aufweist, die im Wesentlichen parallel zu den Achsen der Plattenbohrungen (7) ist und in einem Kompressionsstück (18) bereitgestellt ist, das innerhalb des Einspannvorrichtungsträgers (9) in eine Richtung verschiebbar ist, die an den Längsachsen der Platte (2) und des Einspannvorrichtungsträgers (9) ausgerichtet ist.

13. Anordnung (1) nach Anspruch 12, die ferner einen Aktuator (20) zum Verschieben des Kompressionsstücks (18) innerhalb der Einspannvorrichtung (8) umfasst.

14. Set zur Befestigung einer orthopädischen Platte (2) an einem Knochen, wobei das Set eine Anordnung (1) nach einem der Ansprüche 2 bis 13 umfasst, wobei das Set ferner eines oder mehrere der Folgenden umfasst:
a) ein Verbinder-Befestigungselement (36) zum Verbinden eines bzw. des Verbinderabschnitts (24) der Einspannvorrichtung (8) mit der Platte (2);
b) ein Einführwerkzeug (32) zum Drücken der Platte (2) entlang der Länge des Knochens durch das Gewebe;
c) zumindest ein den Kopf befestigendes Befestigungselement (6b) zum Befestigen eines bzw. des Kopfabschnitts (6) der Platte (2) an dem Knochen;
d) zumindest ein Drückelement zur Anlage an der Oberflächenfläche des Knochens, um einen Knochen in dem Knochenaufnahmeraum (10) in Richtung der Knochenkontaktfläche (5) der länglichen Platte zu drücken;
e) eine Zugschraube mit einem zylindrischen Gewinde zum Ziehen des länglichen Schafts (3) in Richtung des Knochens;
f) eine Kompressionsschraube zum Erstrecken durch eine bzw. die Kompressionsbohrung in einer bzw. der Kompressionsvorrichtung in einen Knochen innerhalb des Knochenaufnahmeraums (10);
g) eine Stabilisationsschraube, die ein paralleles Gewinde zum Stabilisieren eines Bruchs aufweist;
h) eine Ausrichtungsschraube zum Erstrecken durch eine Einspannvorrichtungsbohrung (13) distal zu dem Verbinderabschnitt (24) und durch einen Knochen in dem Knochenaufnahmeraum (10) in eine bzw. die Einführungsabschnittsbohrung (7'), um eine Ausrichtung der länglichen Platte und des Einspannvorrichtungsträgers (9) sicherzustellen;
i) zumindest ein Befestigungselement (100), das einen Spitzenabschnitt zum Erstrecken durch einen Knochen in dem Knochenaufnahmeraum (10) und in eine der Anordnung von Plattenbohrungen (7) zum Befestigen der Platte (2) an dem Knochen aufweist;
j) zumindest eine Bohrführung zum Erstrecken durch eine Einspannvorrichtungsbohrung (13), um einen Knochen innerhalb des Knochenaufnahmeraums (10) zu berühren, wobei die Bohrführung einen Bohrpfad für eine Bohrkrone bereitstellt;
k) zumindest ein verriegelndes Befestigungselement zum Verriegeln eines entsprechenden Bohreinsatzes in dem Einspannvorrichtungsträger (9);
l) zumindest einen Bohranschlag zum Einstellen der axialen Länge des Bohrpfads;
m) zumindest einen Bohreinsatz, wobei der bzw. jeder Bohreinsatz eine Nadelspitze aufweist.

15. Set nach Anspruch 14, umfassend zumindest ein Befestigungselement (100), das einen Spitzenabschnitt zur Erstreckung durch einen Knochen in dem Knochenaufnahmeraum (10) und in eine der Anordnung von Plattenbohrungen (7) zum Befestigen der Platte (2) an dem Knochen aufweist, wobei der Spitzenabschnitt des bzw. jedes Befestigungselements (100) ein entsprechendes sich verjüngendes konisches Gewinde umfasst.

## Revendications

1. Plaque orthopédique (2) destinée à être fixée à un os, la plaque (2) présentant une tige allongée (3) avec une surface de contact d'os (5) configurée pour venir en contact avec l'os et une surface opposée (4), la tige allongée (3) présentant un réseau d'alésages filetés, chaque alésage étant destiné à recevoir une fixation respective (100), **caractérisée en ce qu'**au moins un des alésages filetés du réseau est un alésage de plaque conique (7) s'effilant d'un diamètre maximum au niveau de la surface de contact d'os (5) à un diamètre minimum au niveau de la surface opposée (4).

2. Ensemble (1) permettant de fixer une plaque orthopédique (2) à un os, l'ensemble comprenant :
une plaque orthopédique (2) selon la revendication 1 ; et
un gabarit (8) comprenant un support de gabarit allongé (9), dans lequel la tige allongée (3) de la plaque (2) et le support de gabarit allongé (9) sont alignés sensiblement parallèlement l'un à l'autre afin de définir un espace de réception d'os s'étendant longitudinalement (10) entre la surface de contact d'os (5) de la tige allongée (3) et le support de gabarit.

3. Ensemble (1) selon la revendication 2, dans lequel le gabarit (8) comprend une partie de connecteur (24) connectée entre la plaque (2) et une extrémité axiale du support de gabarit (9), la partie de connecteur (24) étant incurvée ou pliée entre le support de gabarit (9) et la plaque (2) et se trouvant dans un plan qui est sensiblement perpendiculaire à et recoupe transversalement l'axe allongé du support de gabarit (9).

4. Ensemble (1) selon la revendication 2 ou 3, dans lequel la plaque (2) comprend une partie de tête (6) au niveau d'une extrémité axiale de la tige allongée (3), la plaque (2) comprenant au moins un alésage de tête (6a), la partie de tête (6) s'étendant selon un angle par rapport à la tige allongée (3) en s'éloignant du support de gabarit (9).

5. Ensemble (1) selon la revendication 4, dans lequel la partie de tête (6) comprend une partie de réception d'outil (31) destinée à recevoir un outil (32).

6. Ensemble (1) selon l'une quelconque des revendications précédentes, dans lequel le au moins un alésage de plaque conique (7) comprend une partie conique (7a) s'effilant d'un diamètre maximum proximal par rapport à la surface de contact d'os à un diamètre minimum distal par rapport la surface de contact d'os, et une partie cylindrique (7b) proximale par rapport à la surface de contact d'os (5) avec un épaulement entre la partie cylindrique (7b) et la partie conique (7a).

7. Ensemble (1) selon l'une quelconque des revendications précédentes, dans lequel la tige allongée (3) comprend une partie d'insertion conique (22) se terminant au niveau d'une extrémité axiale d'insertion (23).

8. Ensemble (1) selon la revendication 7, dans lequel le réseau d'alésages de plaque (7) comprend un alésage de partie d'insertion (7') au niveau ou à proximité de l'extrémité axiale d'insertion (23), dans lequel l'alésage de partie d'insertion (7') est un alésage fileté cylindrique.

9. Ensemble (1) selon l'une quelconque des revendications précédentes, dans lequel le support de gabarit (9) comprend un réseau d'alésages de gabarit (13) sur une surface interne faisant face à l'espace de réception d'os (10), chaque alésage de gabarit étant aligné axialement avec un alésage respectif du réseau d'alésages de plaque (7).

10. Ensemble (1) selon la revendication 9, dans lequel le support de gabarit (9) comprend en outre un réseau d'alésages transversaux (15) sur une surface latérale du support de gabarit, chaque alésage transversal (15) présentant un axe perpendiculaire aux axes des alésages de gabarit (13) et chaque alésage transversal (15) s'étendant à partir de la surface latérale jusque dans l'un respectif des alésages de gabarit.

11. Ensemble (1) selon l'une quelconque des revendications 3 à 10, dans lequel le support de gabarit (9) comprend un dispositif de compression d'os (16) au niveau d'une extrémité axiale opposée du support de gabarit (9) par rapport à la partie de connecteur (24), le dispositif de compression d'os comprenant un alésage de compression (17) qui peut coulisser vers la partie de connecteur (24).

12. Ensemble (1) selon la revendication 11, dans lequel l'alésage de compression (17) présente un axe sensiblement parallèle aux axes des alésages de plaque (7) et est prévu dans une pièce de compression (18) qui peut coulisser à l'intérieur du support de gabarit (9) dans une direction alignée avec les axes allongés de la plaque (2) et du support de gabarit (9).

13. Ensemble (1) selon la revendication 12, comprenant en outre un actionneur (20) pour faire coulisser la pièce de compression (18) à l'intérieur du gabarit (8).

14. Kit pour fixer une plaque orthopédique (2) à un os, le kit comprenant un ensemble (1) selon l'une quelconque des revendications 2 à 13, comprenant en outre un ou plusieurs parmi :
a) une fixation de connecteur (36) pour connecter une/la partie de connecteur (24) du gabarit (8) à la plaque (2) ;
b) un outil d'insertion (32) pour pousser la plaque (2) à travers le tissu le long de la longueur de l'os ;
c) au moins une fixation de tête (6b) pour fixer une/la partie de tête (6) de la plaque (2) à l'os ;
d) au moins un élément de poussée destiné à une mise en butée avec la surface superficielle de l'os pour pousser un os dans l'espace de réception d'os (10) vers la surface de contact d'os (5) de la plaque allongée ;
e) une vis de traction présentant un filetage cylindrique pour tirer la tige allongée (3) vers l'os ;
f) une vis de compression destiné à s'étendre à travers un/l'alésage de compression dans un/le dispositif de compression d'os jusqu'à l'intérieur de l'espace de réception d'os (10) ;
g) une vis de stabilisation présentant un filetage parallèle pour stabiliser une fracture ;
h) une vis d'alignement destinée à s'étendre à travers un alésage de gabarit (13) distal par rapport à la partie de connecteur (24) et à travers un os dans l'espace de réception d'os (10) dans un/l'alésage de partie d'insertion (7') pour assurer l'alignement de la plaque allongée et du support de gabarit (9) ;
i) au moins une fixation (100) présentant une partie de pointe destinée à s'étendre à travers un os dans l'espace de réception d'os (10) et dans l'un du réseau d'alésages de plaque (7) afin de fixer la plaque (2) à l'os ;
j) au moins un guide de forage destiné à s'étendre à travers un alésage de gabarit (13) pour venir en contact avec un os à l'intérieur de l'espace de réception d'os (10), le guide de forage étant destiné à fournir un trajet de forage pour un foret ;
k) au moins une fixation de verrouillage pour verrouiller un foret respectif dans le support de gabarit (9) ;
l) au moins une butée de forage pour ajuster la longueur axiale du trajet de forage ;
m) au moins un foret, le/chaque foret présentant une pointe d'aiguille.

15. Kit selon la revendication 14, comprenant au moins une fixation (100) présentant une partie de pointe destiné à s'étendre à travers un os dans l'espace de réception d'os (10) et dans l'un du réseau d'alésages de plaque (7) pour fixer la plaque (2) à l'os, dans lequel la partie de pointe de la/chaque fixation (100) comprend un filetage conique effilé respectif.
